# EUROPÄISCHE PATENTSCHRIFT

(11) **EP 1 503 697 B1**
(45) Veröffentlichungstag und Bekanntmachung des Hinweises auf die Patenterteilung: **30.03.2016**
(21) Anmeldenummer: 03747399.8
(22) Anmeldetag: 06.05.2003
(51) Int. Cl.: A61F 2/60, A61F 2/64

(54) **GELENK EINER PROTHESE MIT VIER ACHSBOLZEN**
PROSTHESIS JOINT HAVING FOUR AXIAL PINS
ARTICULATION DE PROTHESE DOTEE DE QUATRE AXES A BOULONS

(30) Priorität: 06.05.2002 DE 20208236 U
(43) Veröffentlichungstag der Anmeldung: 09.02.2005
(73) Patentinhaber: PRO LIMB International Corp., New Taipei City 238 (TW)
(72) Erfinder: Shen, Hsin Fa, Taipei Hsien (TW)
(74) Vertreter: Blaumeier, Jörg
(86) Internationale Anmeldenummer: PCT/DE2003/001457
(87) Internationale Veröffentlichungsnummer: WO 2003/092545

(56) Entgegenhaltungen:
- DE-C- 841 190
- GB-A- 1 533 796
- GB-A- 2 134 392
- US-A- 5 728 173
- US-A1- 2002 026 246

## Beschreibung

### UMFANG DER ERFINDUNG

Die Erfindung betrifft ein Gelenk von Prothese mit vier Achsbolzen, insbesondere ein Gelenk, das den Gehkomfort erhöhen und eine Sturzgefahr vermeiden kann.

### HINTERGRUND DER ERFINDUNG

Die Prothese ist ein künstliches Glied, das zum Ersatz des verlorenen Gliedes dient und die Funktion des verlorenen Gliedes ausüben soll.

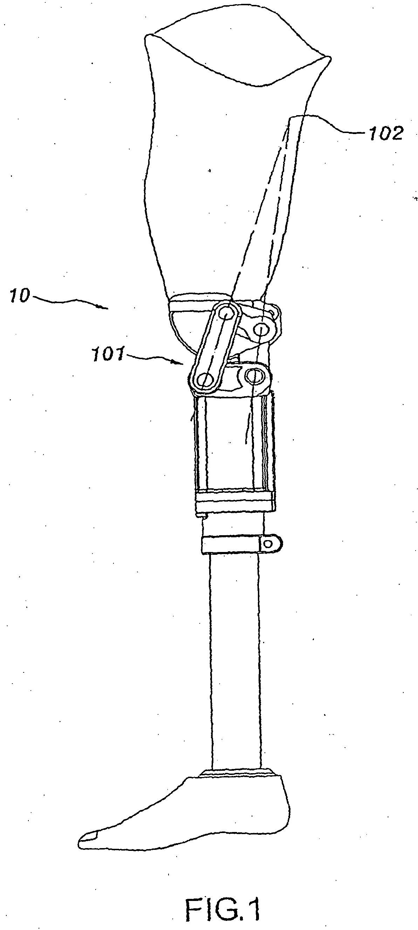

In Figur 1 ist ein herkömmliches Gelenk 10 der Prothese gezeigt, wobei die Achsbolzen 101 eine dreieckige Stützung aufbauen und der Angriffspunkt 102 über dem Kniekopf auf der Spitze des Dreiecks liegt, nämlich in dem verstümmelten Glied.

Da der Angriffspunkt 102 der Achsbolzen 101 über dem Kniekopf liegt, kann das Gelenk 10 leicht gebogen werden, wodurch der Benutzer beim Gehen leicht stürzen kann. Daher droht bei Prothese mit diesem Gelenk 10 eine Sturzgefahr.

Aus diesem Grund hat der Erfinder in Anbetracht der Nachteile herkömmlicher Lösungen, basierend auf langjähriger Erfahrung in diesem Bereich, nach langem Studium, zahlreichen Versuchen und unentwegten

Verbesserungen die vorliegende Erfindung entwickelt.

Aus GB 1 533 796 ist ein Prothesengelenk bekannt. Dieses weist einen Kniekopf und einen Sitz auf, die über eine Gelenkmechanik verbunden sind. Eine erste Achse stellt über zwei obere Schenkel einer Doppelgabel die Verbindung zum Kniekopf her. Eine zweite Achse stellt über zwei Verbindungsplatten eine weitere Verbindung zum Kniekopf her. Eine dritte Achse stellt über die beiden Verbindungsplatten die Verbindung zum Sitz her. Eine vierte Achse schließlich stellt über zwei untere Schenkel der Doppelgabel die Verbindung zum Sitz her. Hierüber wird ein polyzentrischer Gelenkmechanismus gebildet.

### AUFGABE DER ERFINDUNG

Der Erfindung liegt die Aufgabe zugrunde, ein Gelenk von Prothese mit vier Achsbolzen zu schaffen, wobei die vier Achsbolzen ein umgekehrtes Dreieck bilden, wodurch der Angriffspunkt unter dem Sitz zur Verbindung mit dem Unterschenkel liegt, so daß das Gelenk beim Gehen nicht von dem verstümmelten Glied sondern von der Verschiebung des Stützpunktes des Fußes der Prothese kontrolliert wird, weshalb der Gehkomfort erhöht wird und die Sturzgefahr beseitigt wird.

Diese Aufgabe ist erfindungsgemäß dadurch gelöst, daß das Gelenk aus einem Kniekopf, zwei Verbindungsplatten, einer Gabel, einem ersten Achsbolzen, einem zweiten Achsbolzen, einem dritten Achsbolzen, einem vierten Achsbolzen und einem Sitz zur Verbindung mit dem Unterschenkel besteht, wobei der Kniekopf eine erste Achsbohrung und eine zweite Achsbohrung aufweist, wobei der erste Achsbolzen durch die erste Achsbohrung und die oberen Enden der beiden Verbindungsplatten hindurchgeht, während der zweite Achsbolzen durch die zweite Achsbohrung und die Gabel hindurchgeht, wobei die beiden Schenkel der Gabel in den Sitz gesteckt und über den dritten Achsbolzen mit dem Sitz verbunden werden, und wobei der Sitz unten über den vierten Achsbolzen mit den unteren Enden der beiden Verbindungsplatten verbunden wird, wodurch die vier Achsbolzen einen Angriffspunkt haben, der auf der Spitze eines umgekehrten Dreiecks liegt.

Im folgenden wird die Erfindung anhand eines bevorzugten Ausführungsbeispiels und der beigefügten Zeichnungen näher erläutert.

### KURZBESCHREIBUNG DER ZEICHNUNGEN

Figur 1 zeigt eine Seitenansicht der herkömmlichen Lösung.

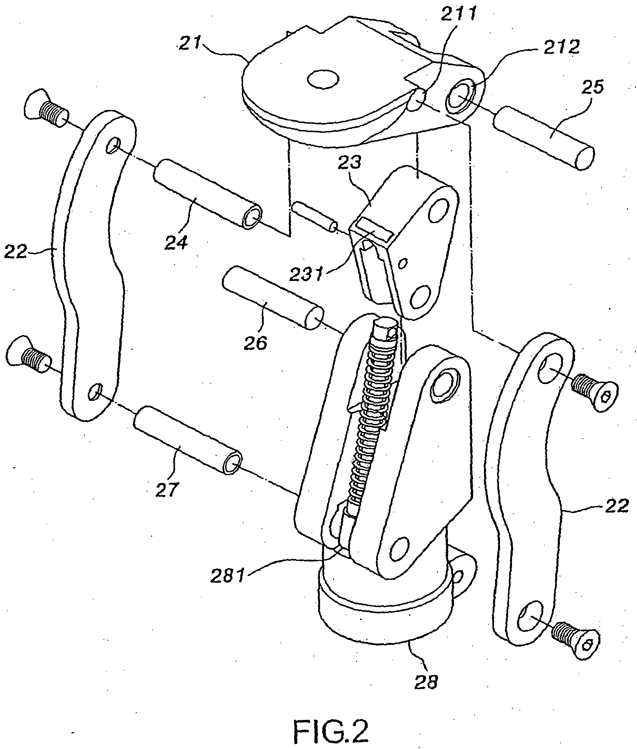

Figur 2 zeigt eine Explosionsdarstellung der Erfindung.
Figur 3 zeigt eine perspektivisch Darstellung der Erfindung.
Figur 4A zeigt eine Seitenansicht der Erfindung beim Einsatz (1).
Figur 4B zeigt eine Seitenansicht der Erfindung beim Einsatz (2).
Figur 4C zeigt eine Seitenansicht der Erfindung beim Einsatz (3).
Figur 4D zeigt eine Seitenansicht der Erfindung beim Einsatz (4).
Figur 4E zeigt eine Seitenansicht der Erfindung beim Einsatz (5).

### DETAILLIERTE BESCHREIBUNG DER

### AUSFÜHRÜNGSBEISPIELE

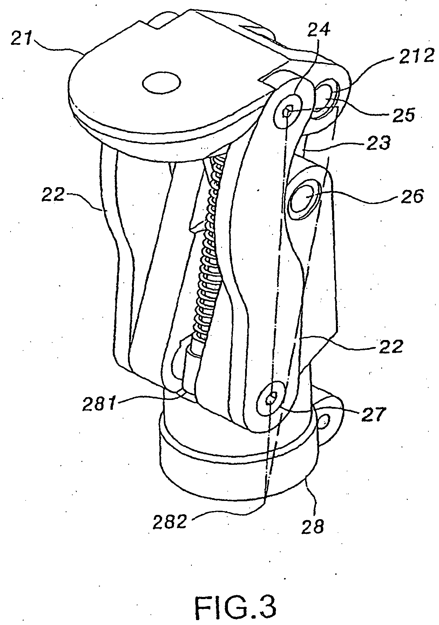

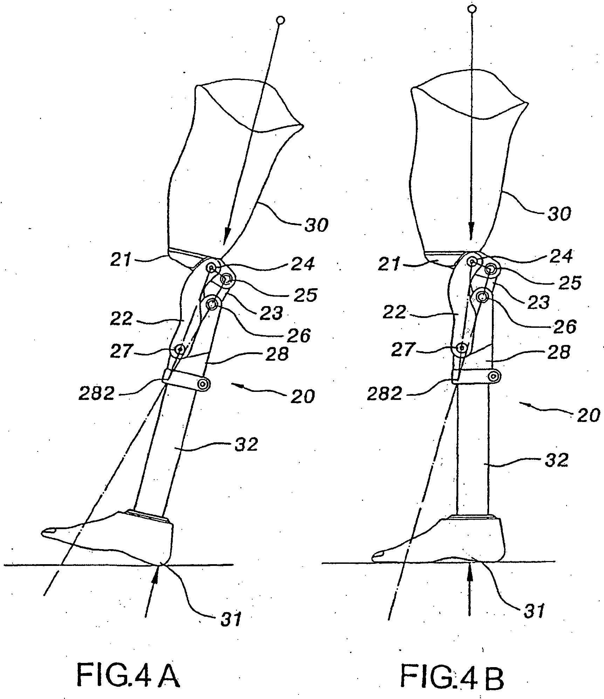

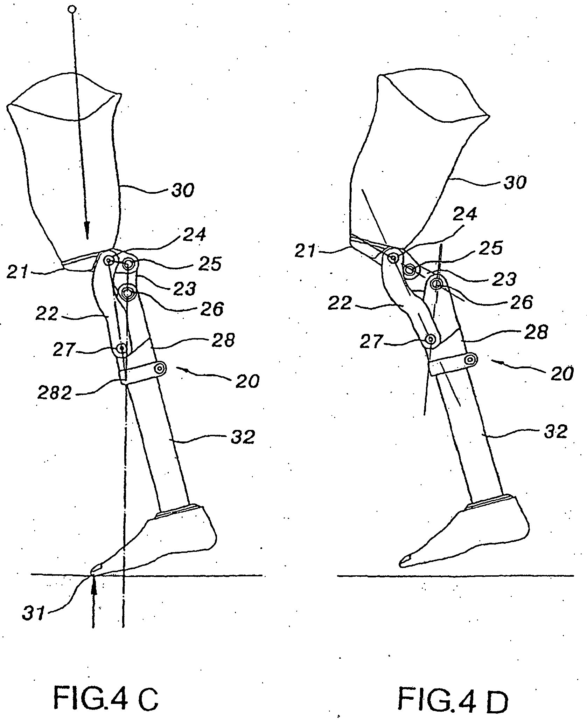

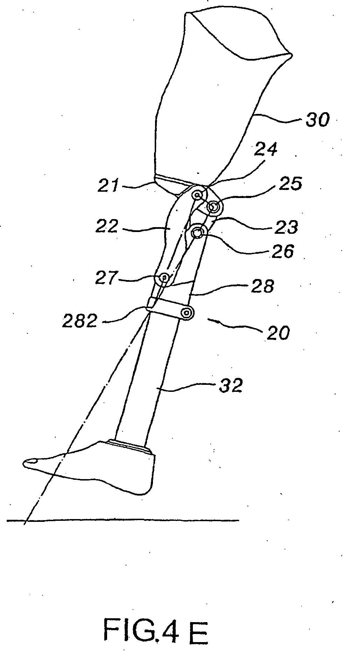

Bezugnehmend auf Figur 3 und 4 besteht die Erfindung im wesentlichen aus einem Kniekopf 21, zwei Verbindungsplatten 22, einer Gabel 23, einem ersten Achsbolzen 24, einem zweiten Achsbolzen 25, einem dritten Achsbolzen 26, einem vierten Achsbolzen 27 und einem Sitz 28 zur Verbindung mit dem Unterschenkel.

Der Kniekopf 21 weist eine erste Achsbohrung 211 und eine zweite Achsbohrung 212 anf, wobei der erste Achsbolzen 24 durch die erste Achsböhrung 211 und die oberen Enden der beiden Verbindungsplatten 22 hindurchgehe während der zweite Achsbolzen 25 durch die zweite Achsbohrung 212 und die Gabel 23 hindurchgeht. Die Gabel 23 kann auf der Stirnseite mit einem Dämppimgselement 231 versehen sein, das zur Vermeidung eines Verschleißes dient. Die beiden Schenkel der Gabel 23 werden in den Sitz 28 gesteckt und über den dritten Achsbolzen 26 mit dem Sitz 28 verbunden. Im Sitz 28 ist ein Federbein 281 vorgesehen, durch das eine Dämpfung erreicht werden kann. Das Federbein 281 dient auch zur Hilfe des Streckens und Ruckstellens des Gelenks. Der Sitz 28 ist unten über den vierten Achsbolzen 27 mit den unteren Enden der beiden Verbindungsplatten 22 verbunden. Die vier Achsbolzen 24, 25, 26 und 27 haben einen Angriffspunkt 282, der auf der Spitze eines umgekehrten Dreiecks liegt (Figur 4).

Wie aus Figur 4A ersichtlich ist, wenn der Benutzer mit der Prothese einen Schritt nach vorne macht und sich mit dem hinteren Ende des Fußes der Prothese auf dem Boden stützt, befindet sich das Gelenk 20 im gestreckten Zustand, in dem der erste, zweite, dritte und vierte Achsbolzen 24, 25, 26, 27 ein umgekehrtes Dreieck bilden und somit eine dreieckige Stützung aufbauen. Dabei kann das Gelenk 20 nicht gebogen werden, egal wie große Kraft das verstümmelte Glied 30 auf das Gelenk 20 ausübt.

Wie aus Figur 4B ersichtlich ist, wenn der Benutzer mit der Prothese steht, befindet sich das Gelenk 20 im vertikalen Zustand, in dem der erste, zweite, dritte und vierte Achsbolzen 24, 25, 26, 27 ebenfalls ein umgekehrtes Dreieck bilden und eine dreieckige Stützung aufbauen. Dabei kann das Gelenk 20 auch nicht gebogen werden.

Wie aus Figur 4C ersichtlich ist, wenn der Benutzer mit dem anderen Bein einen Schritt nach vorne macht, verschieben sich der Stützpunkt des Fußes 31 der Prothese nach dem vorderen Ende und vor dem Angriffspunkt 282 liegt, wodurch die dreieckige Stützung zwischen dem ersten, zweiten, dritten und vierten Achsbolzen 24, 25, 26, 27 abgebaut wird, so daß das Gelenk 20 gebogen werden kann.

Wie aus Figur 4D ersichtlich ist, wenn der Benutzer das verstümmelte Glied 30 nach vorne bewegt, wird die Prothese angehoben.

Wie aus Figur 4E ersichtlich ist, wenn der Benutzer die Prothese anhebt, um einen Schritt nach vorne zu machen, tritt das Gelenk 20 wieder in den gestreckten Zustand ein, wodurch die dreieckige Stützung zwischen dem ersten, zweiten, dritten und vierten Achsbolzen 24, 25, 26, 27 erneut aufgebaut wird. Dabei kann das Gelenk 20 nicht durch das Gewicht des Unterschenkels 32 der Prothese oder die Schwerkraft gebogen werden. Diese dreieckige Stützung zwischen dem ersten, zweiten, dritten und vierten Achsbolzen 24, 25, 26, 27 bleibt bestehen, egal wie lange die Prothese angehoben ist.

Aufgrund den obengenannten Tatsachen entspricht die Erfindung in ihrer Verfügbarkeit, Fortschrittlichkeit und Neuheit vollauf den Anforderungen für ein Gebrauchsmuster.

Die vorstehende Beschreibung stellt nur ein bevorzugtes Ausführungsbeispiel der Erfindung dar und soll nicht als Definition der Grenzen und des Bereiches der Erfindung dienen. Alle gleichwertige Änderungen und Modifikationen gehören zum Schutzbereich dieser Erfindung.

## Patentansprüche

1. Gelenk (20) für eine Prothese, mit einem Kniekopf (21), zwei Verbindungs platten (22), einer Gabel (23), einem ersten Achsbolzen (24), einem zwei ten Achsbolzen (25), einem dritten Achsbolzen (26), einem vierten Achsbolzen (27) und einem Sitz (28) zur Verbindung mit dem Unterschenkel, wobei der Kniekopf (21) eine erste Achsbohrung (211) und eine zweite Achsbohrung (212) aufweist, wobei der erste Achsbolzen (24) durch die erste Achsbohrung (211) und die oberen Enden der beiden Verbindungs platten (22) hindurchgeht, während der zweite Achsbolzen (25) durch die zweite Achsbohrung (212) und die Gabel (23) hindurchgeht, wobei die beiden Schenkel der Gabel (23) in den Sitz (28) gesteckt und über den dritten Achsbolzen (26) mit dem Sitz (28) verbunden sind, und wobei der Sitz (28) unten über den vierten Achsbolzen (27) mit dem unteren Ende der beider Verbindungsplatten (22) verbunden ist, wobei im gestreckten Zustand des Prothesengelenks (20) die gedachten Verbindungslinien, die den ersten Achsbolzen (24) mit dem zweiten Achsbolzen (25), den ersten Achsbolzen (24) mit dem vierten Achsbolzen (27) und den zweiten Achsbolzen (25) mit dem dritten Achsbolzen (26) verbinden, ein umgekehrtes Dreieck aufspannen, und wobei der Angriffspunkt auf der Spitze des umgekehrten Dreiecks liegt, wobei die Spitze unterhalb des vierten Achsbolzens (27) liegt.

2. Gelenk nach Anspruch 1, **dadurch gekennzeichnet, dass** die Gabel (23) auf der Stirnseite mit einem Dämpfungselement (231) versehen ist, das zur Vermeidung eines Verschleißes dient.

3. Gelenk nach Anspruch 1, **dadurch gekennzeichnet, dass** im Sitz (28) ein Federbein (281) vorgesehen ist.

4. Gelenk nach Anspruch 1, **dadurch gekennzeichnet, dass** im Sitz (28) eine Pneumatik oder Hydraulik vorgesehen ist.

## Claims

1. Joint (20) for a prosthesis, with a knee head (21), two connection plates (22), a fork (23), a first axial pin (24), a second axial pin (25), a third axial pin (26), a fourth axial pin (27), and a seat (28) for connection to the lower leg, wherein the knee head (21) has a first axial bore (211) and a second axial bore (212), wherein the first axial pin (24) passes through the first axial bore (211) and the upper ends of the two connection plates (22), while the second axial pin (25) passes through the second axial bore (212) and the fork (23), wherein the two branches of the fork (23) are inserted into the seat (28) and are connected to the seat (28) via the third axial pin (26), and wherein the seat (28) is connected at the bottom to the lower end of the two connection plates (22) via the fourth axial pin (27), wherein, in the extended state of the prosthesis joint (20), the imaginary connection lines connecting the first axial pin (24) to the second axial pin (25), the first axial pin (24) to the fourth axial pin (27) and the second axial pin (25) to the third axial pin (26), span an inverted triangle, and wherein the point of attack lies at the vertex of the inverted triangle, wherein the vertex lies below the fourth axial pin (27).

2. Joint according to Claim 1, **characterized in that** the fork (23) is provided, on the front face, with a damping element (231), which serves to avoid wear.

3. Joint according to Claim 1, **characterized in that** a spring strut (281) is provided in the seat (28).

4. Joint according to Claim 1, **characterized in that** a pneumatic or hydraulic arrangement is provided in the seat (28).

## Revendications

1. Articulation (20) pour une prothèse, comprenant une tête de genou (21), deux plaques de liaison (22), une fourche (23), une première goupille axiale (24), une deuxième goupille axiale (25), une troisième goupille axiale (26), une quatrième goupille axiale (27) et un siège (28) pour la liaison à la jambe, la tête de genou (21) présentant un premier trou axial (211) et un deuxième trou axial (212), la première goupille axiale (24) passant à travers le premier trou axial (211) et les extrémités supérieures des deux plaques de liaison (22), tandis que la deuxième goupille axiale (25) passe à travers le deuxième trou axial (212) et la fourche (23), les deux branches de la fourche (23) étant enfichées dans le siège (28) et étant reliées au siège (28) par le biais de la troisième goupille axiale (26), et le siège (28) étant relié en bas par le biais de la quatrième goupille axiale (27) à l'extrémité inférieure des deux plaques de liaison (22), les lignes de liaison imaginaires qui, dans l'état étiré de l'articulation de prothèse (20), relient la première goupille axiale (24) à la deuxième goupille axiale (25), la première goupille axiale (24) à la quatrième goupille axiale (27) et la deuxième goupille axiale (25) à la troisième goupille axiale (26), formant un triangle inversé et le point d'application étant situé à la pointe du triangle inversé, la pointe étant située en dessous de la quatrième goupille axiale (27).

2. Articulation selon la revendication 1, **caractérisée en ce que** la fourche (23) est pourvue sur le côté frontal d'un élément d'amortissement (231) qui sert à éviter une usure.

3. Articulation selon la revendication 1, **caractérisée en ce qu'**une jambe à ressort (281) est prévue dans le siège (28).

4. Articulation selon la revendication 1, **caractérisée en ce qu'**un système pneumatique ou hydraulique est prévu dans le siège (28).
